**Europäisches Patentamt**

(19) **European Patent Office**

**Office européen des brevets**

(11) Publication number: **0 130 515**
**A2**

# (12) EUROPEAN PATENT APPLICATION

(21) Application number: **84107248.1**

(22) Date of filing: **25.06.84**

(51) Int. Cl.⁴: **C 12 Q 1/68**

(30) Priority: **05.07.83 US 511063**

(43) Date of publication of application: **09.01.85**
**Bulletin 85/2**

(84) Designated Contracting States: **DE FR GB**

(71) Applicant: **Molecular Diagnostics, Inc., 400 Morgan Lane, West Haven, CT.06516 (US)**

(72) Inventor: **Dattagupta, Nanibhushan, 470 Prospect Street, New Haven Ct 06511 (US)**
Inventor: **Rae, Peter M. M., 71 Ingram Street, Hamden Ct 06517 (US)**
Inventor: **Crothers, Donald M., Surrey Drive, Northford Ct 06472 (US)**

(74) Representative: **Adrian, Albert, Dr. et al, c/o BAYER AG Konzernverwaltung RP Patentabteilung, D-5090 Leverkusen 1, Bayerwerk (DE)**

(54) **Testing DNA samples for particular nucleotide sequences.**

(57) A method for determining whether the DNA contained in a test sample includes a particular nucleic acid sequence, comprising the steps of:

a) extracting nucleic acids from the test sample,

b) digesting the extracted nucleic acids with restriction enzyme thereby to cleave the DNA or not at a particular sequence, depending on whether or not a restriction enzyme recognition site is present in the sequence,

c) treating the product of (b) to form single stranded nucleic acids,

d) contacting the single stranded nucleic acids produced in (c) with first and second polynucleotide probes which are complementary to respective first and second portions of said sequence to be detected, the two portions being non-overlapping and immediately adjacent to the restriction site in question. The contact is perfomed under conditions favorable to hybridization of said first and second probes to said sequence to be detected, hybridization with both of said probes being dependent upon whether in step (b) restriction did not occur, said first probe being incorporated with a distinguishable label,

e) separating, by means of said second probe, (i) any resulting dual hybridization product comprising said sequence to be detected hybridized to both said labeled first probe and said second probe, from (ii) any unhybridized and singly-hybridized labeled first probe, and

f) by means of said label detecting any of said separated dual hybridization product which may be present.

The second probe is preferably fixed to a solid support and can be used by mixing the first probe in solution with the unknown and with the solid support carrying the second probe, letting the mass stand under hybridizing conditions, separating the solid support and determining the presence and amount of label attached to the solid support.

ACTORUM AG

The present invention relates to a process and device for determining the presence or absence of particular genetic conditions in samples of plant or animal tissue or fluid, as evidenced by particular alternative nucleic acid constitutions of cells in such material. The condition need not be expressed clinically for its presence or potential to be detected in this test.

Techniques are available for determining genetic conditions such as sickle cell anemia by testing the DNA in tissues, such as white blood cells, of a patient under consideration. Such tests as presently constituted are time-consuming, and require extreme skill in running the test and interpreting the results. They can also be costly, and then can be inaccurate.

It is an object of the present invention to provide a simple, relatively inexpensive, and reliable high resolution test for the presence or absence of particular nucleic acid sequences in test DNA specimens.

These and other objects and advantages are realized in accordance with the present invention pursuant to which there is provided a method for determining whether the nucleic acid in a test sample includes a particular nucleic acid sequence, comprising the steps of:

a)    extracting the nucleic acids from a test sample,

b)    digesting the extracted nucleic acids with a restriction enzyme thereby to effect restriction or not to effect restriction, depending on whether or not the restriction enzyme recognition site is precisely present in a sequence in the test DNA,

MD 201                                                -c-

c) treating the product of (b) to form single-stranded nucleic acids,

d) contacting the single-stranded nucleic acids produced in (c) with first and second polynucleotide probes which are complementary to respective first and second portions of said sequence to be detected, the two portions being non-overlapping and immediately adjacent to the restriction site in question, and such contact being performed under conditions favorable to hybridization of said first and second probes to said sequence to be detected, hybridization with both of said probes to a test molecule being dependent upon whether in step (b) restriction did not occur, said first probe being incorporated with a distinguishable label,

e) separating, by means of said second probe, (i) any resulting dual hybridization product comprising said sequence to be detected hybridized to both said labeled first probe and said second probe, from (ii) any unhybridized and singly hybridized labeled first probe, and

f) by means of said label detecting any of said separated dual hybridization product which may be present.

A suitable kit for running the test is also provided comprising (i) first and second probes each including nucleic acid sequences present in the nucleic acid of a test sample, the first probe carrying a distinguishable label and being soluble in a liquid in which the determination will be run, the second probe being fixed on a solid support, and (ii) a restriction enzyme which will cleave the test sample or fail to depending on the

MD 201

presence or absence of a particular nucleic acid sequence at the point separating sequences complementary to the first and second probes, whereby a positive determination test sample (no cleavage at the point) is capable of hybridizing with both the first and second probes so as thereby to affix the label to the solid support for subsequent reading. The presence of labelling material on the solid support is an index of the extent of dual hybridization.

The first and second probes are themselves formed in a special way. For example, a cloned ß-hemoglobin gene is digested by restriction enzymes which subdivide it into a number of fragments. In the case of detection of the sickle cell defect, the two of interest are a 340 bp (base pair) unit and a 201 bp unit. The fragments are separated from one another by subcloning, and one of them, the 340 bp unit fragment, is fixed to a solid support such as a nitrocellulose sheet or disc, constituting the fixed probe. The other fragment is labeled either with a radioactive group, or a chemically detectable group such as a modified base or a visually detectable group such as one which fluoresces or has characteristic absorbance of ultraviolet or infrared light.

A key to the procedure, in the difference between an affected and unaffected test sample, is that in one of them, upon treatment with a restriction enzyme, there will be fragments longer than each probe so as to be capable of hybridizing with both the fixed probe and the labeled probe whereas the other will not have such longer units, so it will be incapable of hybridizing with both.

MD 201

The dual hybridization is the key to attaching the label to the solid support via one test sample but not the other, such support being what is ultimately analyzed for the presence of label.

With sickle cell anemia as an example, a normal hemoglobin gene can be used to make probes of 340 and 201 bp, and sickle cell anemia hemoglobin DNA, upon digestion, will have a long fragment which covers the two and can dually hybridize, while similarly treated normal DNA will have no such fragment.

The manner of enzymatically digesting the DNA material for producing the probes and/or treating the test samples is known in the art, as are methods for the various separations.

The invention has been particularly described with reference to sickle cell anemia, but it is applicable as well to any other genetic disorders whose presence can be associated with a particular molecular alteration of DNA. One need only find a particular restriction enzyme which will degrade only one of an affected or unaffected gene to form the fragments needed for making the probes which can then distinguish between affected and unaffected test samples pretreated in such manner as one or the other will form a long fragment capable of dually hybridizing with both probes.

The invention will be further described with reference to the accompanying drawings, wherein:

Fig. 1 is a graphic representation of the hemoglobin gene, the preparation of first and second probes and the digestion products of normal and sickle cell DNA; and

MD 201

Fig. 2 is a flow sheet of the method of the invention involving dual hybridization with first and second probes and an unknown DNA.

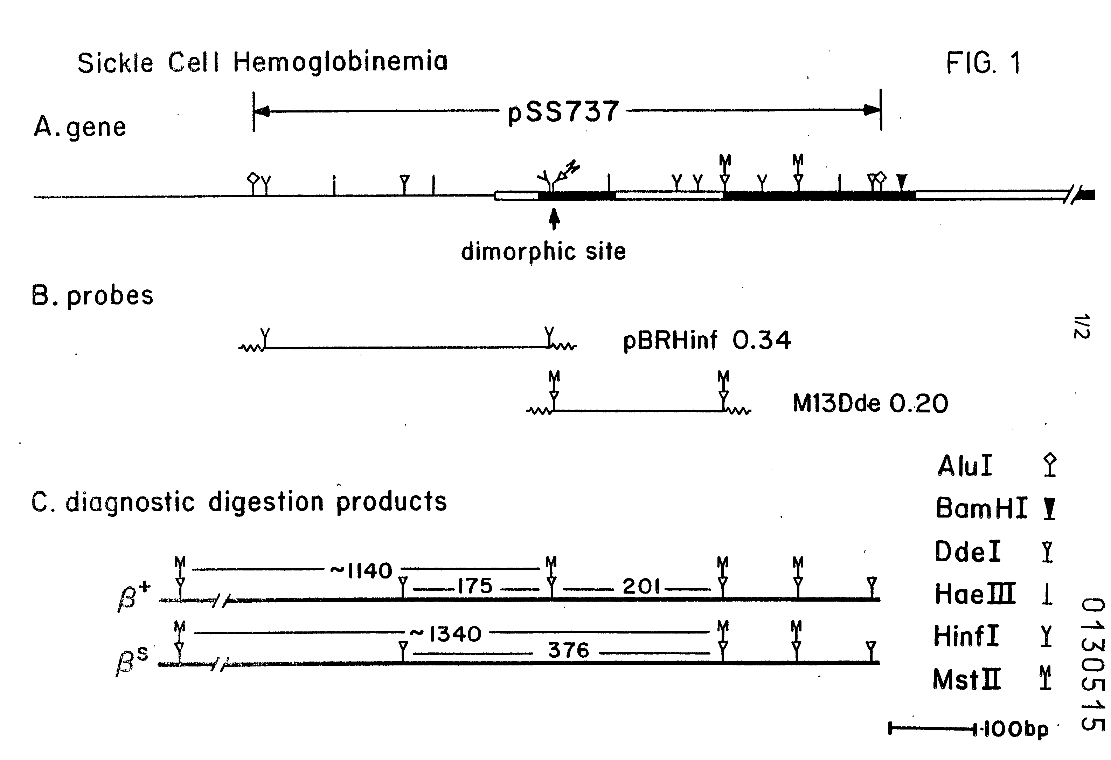

Referring now more particularly to the drawings, in Fig. 1, A is a map of the beginning of the gene encoding ß-hemoglobin, together with flanking DNA that is not expressed (the thin line to the left). The thick portion defines DNA that is transcribed into RNA that is the precursor of messenger RNA that is translated into ß-hemoglobin, and this continues off the figure to the right. Filled-in areas are those that actually encode the amino acid sequence of the protein; the three clear areas are, from left to right, an untranslated leader sequence that is present in the mRNA, a short intervening sequence, and a long intervening sequence. The two intervening sequences are present in the RNA precursor to globin mRNA, but are absent from the mRNA itself; they are removed in intramolecular splicing events that are part of the mRNA maturation process. Symbols above the lines indicate restriction enzyme cleavage sites, and the Dde I/Mst II site specifically absent from a sickle cell gene is indicated. The 0.74 kb Alu I segment represented in the cloned plasmid pSS737 is also indicated.

B shows examples of subcloned segments of the ß-globin gene that can be used as probes in the dual hybridization test. There is a Hinf I site 5 base pairs upstream of the dimorphic DdeI/Mst II site, and this is one end of a 0.34 kb Hinf I segment that has been cloned via a plasmid vector (pBR322). The dimorphic Dde I site itself is one end of a 0.20 kb Dde I segment that was

MD 201

subcloned via a phage vector (M13 MP8) that shares no sequences with pBR. The reciprocal clonings have also been done. In the drawing, flat lines are the cloned human DNA segments, and jagged lines are adjacent portions of vector sequence. The reason that adjacent Dde I segments were not simply subcloned is that the 0.175 kb Dde I segment (see C below) is unnecessarily short. The reason that adjacent Mst II segments (one of which is 1.14 kb long; see C) were not subcloned is that DNA treated with Mst II cannot be ligated.

C is a comparison of the relevant products of Dde I or Mst II digestion of human DNA that contains the normal ß-globin allele, with DNA that contains the sickle cell allele. DNA from persons having the sickle cell trait (+/s) will have both sets of DNA segments.

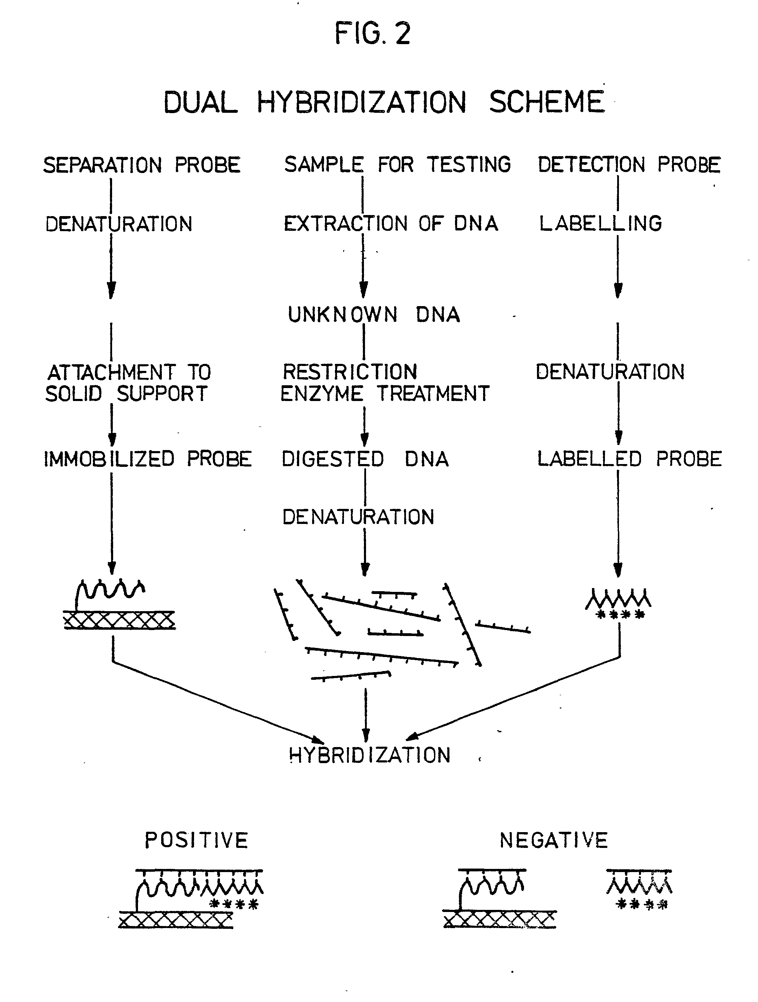

In Fig. 2 at the left and right one starts with separation and detection probes produced as hereinabove described. The denatured separation probe is attached to a solid support. The detection probe is labeled and denatured.

The unknown specimen is treated to extract its DNA which is digested and denatured. Then the probes and treated DNA are mixed under hybridization conditions. The solid with any dually hybridized material attached thereto is then assayed for the label.

The invention will be further described in the following illustrative example wherein all parts are by weight unless otherwise expressed.

MD 201

EXAMPLE

APPLICATION OF THE "DUAL HYBRIDIZATION" TEST TO SICKLE CELL HEMOGLOBINEMIA

1.    Required DNAs:      For a simple example of the utility of dual hybridization, one needs only the plasmid pBR322ßPst or the plasmid pSS737 (both obtained from Dr. John Wilson of the Medical College of Georgia, and described in Geever, et al (1981) Proc. National Academy of Science U.S.A. 78, 5081-5085, or any other of the several cloned human ß-hemoglobin genes that are available, together with segments subcloned thereform (see item 2, below). For an example of its applicability to sickle cell hemoglobinemia (and its extensibility to other disorders that involve restriction site polymorphism), one also needs DNA from +/+ and s/s individuals.

2.    Subcloning the probes:    The plasmid pSS737 contains a 0.74 kb Pst I segment of human DNA that includes the ß-hemoglobin gene (Fig. 1). The restriction site of interest in the clones is a DdeI/Mst II site involving codons 5-7 of the gene, as in the sickle cell allele there is a single A to T change that alters the enzymes' recognition/cleavage site (CTNAG for Dde I; CCTNAGG for Mst II). The aim of subcloning is to dissect the cloned wild-type gene at this point to make two probes having adjacent (or, in general, nearly adjacent) and non-overlapping sequences. One probe, designated the separation probe, will be matrix-bound and single-stranded, and will immobilize an unknown DNA by hybridization. The other probe, designated the detector probe,

MD 201

will be labelled and also single-stranded, and will hybridize with the unknown DNA in solution. The unknown DNA will have been treated with the restriction enzymes Dde I and/or Mst II, and the question is whether or not the detector probe can become immobilized via a bridge to the separation probe, the bridge being provided by the unknown DNA. If the unknown DNA is wild-type with respect to the ß-globin gene, Dde I or Mst II digestion will obviate such a linkage. However, a sickle cell allele will be insensitive to the enzymes at the relevant site, so genomic DNA that is s/s or s/+ will allow the immobilization of some detector probe (see items 3 and 4 for the digestion of genomic DNA and the labelling of detector probe).

The subcloning proceeds as follows: The plasmid pSS737 is purified and one sample is digested to completion with Dde I; another sample is digested with Hinf I (see Fig. 1). The resulting DNA segments are separated according to size by electrophoresis in a preparative, low melting temperature agarose gel. The gel is stained with ethidium bromide to allow visualization by UV transillumination, and DNA bands of 0.34 kb from the Hinf I digest, and 0.20 kb from the Dde I digest, are excised. Agarose in the gel slices is melted, and DNA is purified by phenol extraction and ethanol precipitation. The DNAs are redissolved in water. At this point, the 0.34 kb Hinf I digestion products have the structure

$$5' \text{ ANTC......G}$$
$$\text{G......CTNA } 5'$$

and the 0.20 kb

MD 201

Dde I digestion products have the structure

```
5' TNAGG....CC
        CC... GGANT 5'
```

It is desirable to clone the segments in such a way that the respective Hinf I and Dde I/Mst II sites at the termini are retained, and the practical way to do this is to blunt-end ligate the segments to a vector that has been linearized with an enzyme that gives ends that, upon fusion with a segment to be cloned, contribute to the restoration of the enzyme cleavage site. In particular, Hinf I segments can be inserted into pBR322 that has been digested with Cla I to restore the Hinf I cleavage sites; Dde I (Mst II) segments can be inserted into pBR322 that has been digested with Nae I, or into phage M 13 MP8 DNA that has been digested with Sma I.

Cla I cleaves pBR322 once at the site

```
...AT CGAT....
....TAGC TA...
```

The filling-in of overlapping ends by the large (Klenow) fragment of E. coli DNA polymerase produces

```
...ATCG    CGAT...
...TAGC    GCTA...
```

The filling-in of ends of the Hinf I segment ro be sub-cloned produces

```
ANTC......GANT
TNAG......CTNA
```

and ligation of this with the vector results in a sequence,

```
...ATCGANTC......GANTCGAT...
...TAGCTNAG......CTNAGCTA...
```

that retains the Hinf I sites (overlines).

MD 201

The 0.20 kb Dde I (Mst II) segment of the ß-globin gene is to be ligated to the ends of a vector linearized with an enzyme that gives the following ends:

```
...CC   GG...
...GG   CC...
```

One such enzyme is Sma I (CCC GGG), and this can be used for cloning into the E. Coli phage M13 MP8, which has a single Sma I site. Another such enzyme is Nae I (GCC GGC), which cleaves pBR322 four times, at 401, at 1282, and at two other sites in between. The plasmid is left intact between 1282 and 401, and the only function disrupted is the ability to confer tetracycline resistance. For the cloning of the 201 pb Dde I segment, vector preparation involves the digestion of pBR322 with Nae I and a treatment of the products with alkaline phosphatase to remove terminal phosphates (this discourages simple vector circularization during subsequent ligation). The large vector segment is purified by preparative gel electrophoresis. To accomplish the blunt-end ligation, the ends of the ß-globin Dde I segment are filled in to make them flush. As suggested above, this is done by treating the segments with the Kleno fragment of E. coli DNA polymerase plus the four deoxyribonucleoside triphosphates. A mixture of these segments and the Nae I treated vector is incubated with $T_4$ DNA ligase plus ATP, then is used to transform bacterial cells made competent to take up naked DNA. The cells are plated on medium containing an antibiotic, resistance to which is conferred by plasmid that is taken up by cells (e.g., ampicillin resistance in the case of pBR322).

MD 201

Colonies of individual transformants are screened for plasmids of interest by growing some cells in a small volume of liquid, then gently lysing the cells to release plasmid DNA. The DNA is purified, treated with the indicated restriction enzymes that give segments diagnostic for the presence in the plasmid of the desired insert, then the DNA is displayed according to size in an agarose gel. Once appropriate recombinant plasmids have been identified, colonies from which they originated are used to seed large cultures from which several hundred micrograms of plasmid are prepared. The colonies are also maintained on plates, and stored frozen for later use.

In the specific case of the development of plasmid probes for the detection of the sickle cell variant of the ß-hemoglobin gene, one plasmid will contain the 0.34 kb Hinf I segment, and another contains the adjacent 0.20 kb Dde I segment. Because there is at least the possibility that in the dual hybridization scheme hybridization of vectors could give false positive results, it is desirable that the two probes be carried in unrelated vectors. This is most cleanly done using an E. coli host/vector system for one probe, and a B. subtilis host/vector system for the other. Alternatively, one probe can be in an E. coli plasmid such as pBR322, and the other can be in an E. coli phage, such as M13.

3.  **Immobilizing the separation probe and labelling**
    **the detector probe:**    . Plasmid containing the separation probe, e.g., a subclone of the 0.34 kb Hinf segment of pSS737 /Fig. 2) is treated with 0.1 m NaOH

MD 201

for 5 minutes, then chilled in ice. The sample is neutralized with an equal volume of 0.1 N HCl, 0.9 M NaCl, 0.09 M Na citrate, then filtered under mild aspiration through a nitrocellulose filter (e.g., BA 85 from Schleicher and Schuell) that had been presoaked in 0.9 M NaCl, 0.09 M Na citrate (6 x SSC; 1 x Standard Saline Citrate = 0.15 M NaCl, 0.015 M Na citrate). The filter is then washed with 6 x SSC, then 70 % ethanol, and baked under vacuum at 80°C for a few hours, or with no vacuum at 65°C overnight. At this point, the filter is ready for hybridization procedures, but it can be stored dry for many months.

The purpose of the alkali treatment of plasmid is to denature the DNA. This renders it both capable of binding to nitrocellulose (native DNA does not) and available for subsequent hybridization with other single-stranded DNA. Neutralization of the denatured DNA solution with acid and the addition of salt (as 6 x SSC) facilitate the binding of denatured DNA to nitrocellulose, and the low temperature inhibits reannealing of the plasmid while it is being loaded onto the nitrocellulose. Baking of the filter finally immobilizes the DNA.

Labelling of the detector probe with, for example, $^{32}$P can be accomplished in several ways, all of wich are standard methods: The ends of a plasmid that has been made linear by the action of a restriction endonuclease can be labelled either by a reaction in which polynucleotide kinase adds the terminal phosphate of $\gamma$-$^{32}$P-ATP to the 5' end of DNA molecules; by the filling in of recessed 3' ends that are generated by some restriction enzymes,

MD 201

through the use of the large (Klenow) fragment of E. coli DNA polymerase and $\alpha^{32}$P-labelled deoxyribonucleoside triphosphates; or by the 3' terminal addition of labelled nucleotides through the action of terminal addition of labelled nucleotides through the action of terminal deoxynucleotidyl transferase. More extensive labelling of a probe can be accomplished using phage $T_4$DNA polymerase, or by strand replacement DNA synthesis ("nick translation") using E. coli DNA polymerase, labelled triphosphates, and plasmid that has been randomly nicked by deoxyribonuclease I to produce numerous synthesis primer sites. Other labelling schemes that do not involve radioactivity are also possible.

4.    Preparation of samples of unknown DNA:   For the simplest example of the utility of dual hybridization, use can be made of the plasmid pBR322ßPst or the plasmid pSS737 (Fig. 2) as the unknown as long as it can be assured that the subcloned detector probe does not hybridize with the immobile selector subclone in the absence of the "unknown" (for the purpose of the model, it does not matter that the detector can hybridize with the "unknown" through both globin segment homology and vector homology). Since the Pst and the SS737 segments are already in pBR322, the best way to construct the two probes is to put the selector probe (the 0.20 kb Dde I segment, as discussed in (2) into a B. subtilis vector, or into the E. coli phage M13, and the detector probe into pBR322. To make pBR322 Pst a useful unknown, one

MD 201

aliquot is digested with Mst II and/or Dde I, and this represents DNA containing the wild-type globin gene; pSS737 can be treated with Dde I. A second aliquot of plasmid is linearized by an enzyme that cleaves outside of the region in question, but ideally a segment is generated that has a length not too much greater than the 0.38 kb Dde I segment, nor the 1.34 kb Mst II segment that results from digestion of CNA containing the sickle cell allele of the globin gene. Such a mimic of sickle cell DNA could result from the digestion of pBR322βPst with Alu I, giving a segment of 0.74 kb with the dimorphic Dde I/Mst II site in the middle. The same result obtains if pSS737 is treated with EcoRI, because SS737 has the identical Alu I segment, inserted into the EcoRI site of pBR322 via synthetic EcoRI linkers.

For the example in which genomic DNA from s/s and +/+ is used, the DNA is most easily prepared from 10 to 50 ml of blood with separation from RBC's, lysing of white cells in high salt and detergent, extraction of nucleic acids using phenol and an ethanol precipitation, as follows:

RAPID PREPARATION OF DNA

i). To a sample of blood (or of amniotic fluid, urine or a dispersed biopsy) add 1/4 volume of 0.25 M Tris-HCl, pH 7.8, 0.5 M Na$_2$EDTA, 1 % Nonidet P-40. Mix gently but thoroughly.

ii). Collect the crude preparation of nuclei by low-speed centrifugation in a conical tube.

MD 201

Suspend nuclei in 0.5 ml 0.05 M Tris, 0.1 M EDTA. Transfer to a 5 ml tube.

iii). Add 0.5 ml 4 M NaCl, 2 % Sarkosyl, and mix vigorously and thoroughly. Add 1 ml phenol/chloroform $\overline{/1}$:1, phenol (satd. w/tris-EDTA) and chloroform$\overline{m/}$, and emulsify. Continue vigorous shaking for 5 minutes then centrifuge at high speed.

iv). Collect the (upper) aqueous phase, leaving the organic phase and the band of denatured protein undisturbed. Transfer the solution of another 5 ml tube, then add 2.5 ml 95 % ethanol. Mix thoroughly, then centrifuge at high speed.

v). Decant the supernatant, then add 3 ml 70 % ethanol to tube. Mix vigorously (vortex) to suspend the pellet, then centrifuge at high speed. Decant the ethanol, then repeat the 70 % ethanol wash. After centrifugation and decantation, add 3 ml 95 % ethanol, Mix, then centrifuge at high speed. Decant the ethanol, then dry the tube.

vi). Dissolve the pellet in 0.1 ml restriction enzyme digestion buffer, then add 0.01 ml restriction enzyme. Incubate at 37°C for 1 hr. or more.

A ribonuclease plus amylase step can also be included and, to ensure the purity of DNA, nucleic acids can be centrifuged in a CsCl buoyant density equilibrium gradient. The DNA is collected from gradient fractions

MD 201

by precipitation with three volumes of 70 % ethanol, with the precipitate being washed with 70 % and 95 % ethanol. The precipitate is dried, then DNA is dissolved in TE (10 mM Tris.HCl, pH 7.6, 1 mM $Na_2EDTA$) or water.

In using genomic CNA as the unknown, as will be the case in the best example and in the field, it is important that restriction enzyme digestions reach or closely approach completion. In particular, the DdeI/Mst II site present in wild-type DNA, but absent in the sickle cell gene, has to be cleaved in samples of DNA lacking the sickle cell allele. Because Mst II that is commercially available is not highly active, special care must be taken to ensure that digestions are as complete as possible. Such care can involve the use of an order of magnitude more enzyme than is theoretically required. It can also involve, in the special case of sickle cell, the digestion of DNA with excesses of both Mst II and Dde I (in this case, sickle cell DNA segments of interest will be 0.38 kb long rather than 1.34 kb long, but this does not matter very much). Increasing the length of time of digestion should not be relied upon, since many enzymes become inactive within an hour or less of dilution into a digestion mixture.

5.  Hybridization:     As the hybridization procedure involves single-stranded DNAs, the nitrocellulose filter upon which the selector probe is immobilized must be pretreated so that the unknown DNA and the detector probe do not bind to it indiscriminately. Such treatment

MD 201

commonly involves saturating available sites on the filter with protein and polysaccharide in a mixture known as Denhardt's solution (0.2 % each of bovine serum albumin, ficoll and polyvinylpyrrolidone in water), in which, along with some salt and buffer (e.g., 6 x SSC, 0.1 M Tris, pH 8), a filter is soaked for a few hours at the temperature to be used for hybridization (e.g., 65°C). The presoak solution is then replaced with hybridization medium that includes denatured sample (unknown) DNA and denatured detector probe, and DNA annealing is allowed to proceed for a few hours. Two representative hybridization conditions are: (i) 6 x SSC, 0.1 M Tris, pH 8, 65°C, the inclusion of Denhardt's solution being optional; (ii) 4 x SSC, 40 % formamide, 40°C, ± Denhardt's solution. To minimize the volume of liquid needed, and to prevent evaporation, hybridizations are often done in plastic bags that have been pressed flat and sealed.

After hybridization, DNAs that have not been faithfully base paired to the selector probe are washed from the filter by a series of filter soakings in solutions that demand extensive annealing for hybrid stability to be maintained. For example, the filter is soaked in two changes of a large volume of 0.2 x SSC at 65°C (at which low salt concentration poorly base paired hybrids will dissociate), then in two changes of a large volume of 0.2 x SSC at room temperature. The filter is then dried by blotting on filter paper. If the detector probe is labelled with $^{32}$P, the filter is subjected to autoradiography by overlaying the filter with a sheet of

MD 201

X-ray film which is developed after a time; alternatively, filter discs or portions of a filter sheet can be assayed for radioactivity by immersing them in water and measuring Cerenkov radiation in a liquid scintillation spectrometer.

6. Explanations of procedures in part 4: With reference to the foregoing procedures in Part 4, the purpose of the addition of Tris, EDTA and Nonidet in (i) to a sample of cells in fluid is to lyse cells, but not nuclei, and to inhibit deoxyribonucleases while so doing. Tris is the buffer tris(hydroxymethyl)aminomethane; $Na_2$EDTA is disodium ethylenediamine tetraacetate, a chelator of divalent cations that are required for DNase activity; nonionic detergents such as NP-40 (or Triton X-100) solubilize cell membranes but do not significantly affect cell nuclei.

The result of this step is that cells, particularly RBC's, are disrupted, with virtually only nuclei remaining intact. The purpose is to condition the sample so that nearly all cell material but nuclei will remain in the supernatent during the centrifugation at low speed in step (ii).

The purpose of step (ii) is to concentrate nuclei by low speed centrifugation (e.g., 1 kg for 5-10 min.), and to separate them from the rest of cell material and the fluid by simply pouring off the supernatant. The nuclei are suspended in the Tris-EDTA of step (i) without the detergent in order to transfer them to an extraction tube for step (iii). The transfer can be

MD 201

avoided by using a centrifuge tube with a bottom which is open and threaded to accept a 5 ml cappable extraction tube which itself can sustain high-speed centrifugation in steps (iii) to (v).

If the pelleted nuclei are sticky, they can be resuspended by including the polyanions spermine and/or spermidine in the buffer (and optionally also in the 4x buffer used in step (i)).

The purpose of step (iii) is to dissociate nucleo-protein complexes with high salt, to denature protein with the ionic detergent Sarkosyl (Na lauryl sarcosinate, which has the advantage over SDS that it is soluble in high salt and in the cold), and to remove proteins from the solution by partitioning them into the organic solvent phenol or precipitating them with phenol and chloroform.

Upon centrifugation, the emulsion produced by shaking is separated into three phases: a lower organic phase, a band of denatured protein, and an upper aqueous phase that contains nucleic acids and any carbohydrates (mainly glycogen) that may have accumulated in the cells.

The purpose of step (iv) is to recover carefully the aqueous phase, then precipitate nucleic acids by making the solution about 70 % in ethanol. If collection of the aqueous phase, by pipetting, is difficult due to viscosity created by the presence of high molecular weight DNA, vigorous shaking in step (iii) will sufficiently reduce the size of DNA or it may be necessary to introduce some deliberate shearing of the DNA /as by altering step (iii) so that suspended nuclei are treated

MD 201

first only with 2 M NaCl in a syringe, forced through a 27 ga. needle into the extraction tube to shear the DNA, and then subjected to Sarkosyl and phenol/chloroform[7].

The purpose of the ethanol washes in step (v) is to remove residual solvent and salts from the precipitate of nucleic acids. After the 95 % ethanol wash, the tube should be dried short of the point where the pellet is completely dry. Damp DNA dissolves more readily than dry DNA. One wants to remove practically all of the ethanol so that it doesn't interfere in step (vi), but not so much liquid that the DNA is totally dry. The tube may be drained inverted for some minutes, then wiped inside with a Q-tip except in the immediate vicinity of the pellet.

The purpose of step (vi) is to dissolve the DNA in a buffer that is suitable for the action of a restriction endonuclease, then to digest the DNA with the enzyme. Restriction enzyme buffers generally contain millimolar amounts of Tris buffer, $Mf^{++}$, and mercaptoethanol, and concentrations of salt as high as 0.15 M. Also, it is sometimes desirable to include bovine serum albumin to help stabilize the enzyme. Because salts, and the presence of $Mg^{++}$ in particular, will inhibit the dissolution of DNA, it is advantageous to dissolve the DNA in water before adding a fractional volume of concentrated buffer/ salts. For example, the DNA pellet can be dissolved in 0.08 ml of $H_2O$, and to this can be added 0.01 ml of 10x buffer/salts and 0.01 ml of enzyme.

MD 201

It will be understood that the specification and examples are illustrative but not limitative of the present invention and that other embodiments within the spirit and scope of the invention will suggest themselves to those skilled in the art.

MD 201

CLAIMS:

1.    A method for determining whether the DNA contained in a test sample includes a particular nucleic acid sequence, characterized in that it comprises the steps of:

a) extracting nucleic acids from the test sample,

b) digesting the extracted nucleic acids with restriction enzyme thereby to cleave the DNA or not at a particular sequence, depending on whether or not a restriction enzyme recognition site is present in the sequence,

c) treating the product of (b) to form single-stranded nucleic acids,

d) contacting the single-stranded nucleic acids produced in (c) with first and second polynucleotide probes which are complementary to respective first and second portions of said sequence to be detected, the two portions being non-overlapping and immediately adjacent to the restriction site in question, such contact being performed under conditions favorable to hybridization of said first and second probes to said sequence to be detected, hybridization with both of said probes being dependent upon whether in step (b) restriction did not occur, said first probe being incorporated with a distinguishable label,

e) separating, by means of said second probe, (i) any resulting dual hybridization product comprising said sequence to be detected hybridized to both said labeled first probe and said second probe, from (ii) any unhybridized and single hybridized labeled first probe, and

f) by means of said label detecting any of said separated dual hybridization product which may be present.

MD 201

2.    A method according to claim 1, characterized in that the second probe is in a form which enables the hybridization product of the first probe and said sequence to be detected to be separated from unhybridized and singly hybridized first probe.

3.    A method according to claim 2, characterized in that the second probe is fixed to a solid support.

4.    A method according to any of claims 1 to 3, characterized in that each of the probes is independently produced by sub-cloning in a different host vector system.

5.    A method according to any of claims 1 to 4, characterized in that the first and second probes are obtained by digestion of a cloned nucleic acid containing material identical with that of the test sample, digestion producing two nucleic acid fragments, separating the two fragments so that one becomes the second probe, and applying a distinguishable label to that fragment which becomes the first probe, application of the label being effected either before or after digestion or separation.

6.    A method according to claim 5, characterized in that the digestion is effected with restriction enzymes.

7.    A method according to claim 5 or 6, characterized in that the first and second probes are produced by digestion of a hemoglobin gene, the test sample being the hemoglobin gene of a patient being tested for sickle cell anemia.

8.    A method according to claim 7, characterized in that the enzymatic digestion of the test sample is such that it will leave sickle cell anemia nucleic acid

MD 201

of a length which can hybridize with both probes.

9. A method according to claim 8, characterized in that the first and second probes are produced by digestion of a normal hemoglobin gene, hybridization of the digested test sample to both indicating the presence therein of sickle cell anemia nucleic acid.

10. A kit for determining whether the nucleic acid contained in a test sample includes a particular nucleic acid sequence, comprising (1) first and second probes each including nucleic acid sequences present in the nucleic acid of a test sample, the first probe carrying a distinguishable label and being soluble in a liquid in which the determination will be run, the second probe being fixed on a solid support, and (2) a restriction enzyme which will cleave the test sample or fail to depending on the presence or absence of a particular nucleic acid sequence, whereby a positive determination test sample is capable of hybridizing with both the first and second probes so as thereby to affix the label to the solid support for subsequent reading.

MD 201

Sickle Cell Hemoglobinemia

A. gene

pSS737

dimorphic site

B. probes

pBRHinf 0.34

M13Dde 0.20

C. diagnostic digestion products

$\beta^+$

~1140

175

201

$\beta^s$

~1340

376

AluI

BamHI

DdeI

HaeIII

HinfI

MstII

├────────┤ 100bp

FIG. 1

# FIG. 2

## DUAL HYBRIDIZATION SCHEME

SEPARATION PROBE     SAMPLE FOR TESTING     DETECTION PROBE

DENATURATION       EXTRACTION OF DNA      LABELLING

UNKNOWN DNA

ATTACHMENT TO        RESTRICTION           DENATURATION
SOLID SUPPORT         ENZYME TREATMENT

IMMOBILIZED PROBE     DIGESTED DNA        LABELLED PROBE

DENATURATION

HYBRIDIZATION

POSITIVE                     NEGATIVE